(19) Europäisches Patentamt
European Patent Office
Office européen des brevets

(11) Publication number: **0 108 285 B1**

(12) **EUROPEAN PATENT SPECIFICATION**

(45) Date of publication of patent specification: **20.04.88**

(51) Int. Cl.[4]: **C 07 D 473/32,** C 07 D 473/40, A 61 K 31/52 // C07D473/24

(21) Application number: **83110227.2**

(22) Date of filing: **13.10.83**

(54) **Antiviral purine derivatives.**

(30) Priority: **14.10.82 US 434393**
**14.10.82 US 434384**
**14.10.82 US 434394**
**14.10.82 US 434395**
**28.07.83 GB 8320309**

(43) Date of publication of application:
**16.05.84 Bulletin 84/20**

(45) Publication of the grant of the patent:
**20.04.88 Bulletin 88/16**

(84) Designated Contracting States:
**AT BE CH DE FR GB IT LI LU NL SE**

(56) References cited:
**DE-A-2 539 963**

(73) Proprietor: **THE WELLCOME FOUNDATION LIMITED**
**183-193 Euston Road**
**London NW1 2BP (GB)**

(72) Inventor: **Schaeffer, Howard John**
**4112 Oak Park Road**
**Raleigh, NC 27612 (US)**
Inventor: **Krenitsky, Thomas Anthony**
**106 Laurel Hill Road**
**Chapel Hill, NC 27514 (US)**
Inventor: **Beauchamp, Lilia Marie**
**3007 Wycliff Road**
**Raleigh, NC 27607 (US)**

(74) Representative: **Sandmair, Kurt, Dr. et al**
**Patentanwälte Schwabe, Sandmair, Marx**
**Stuntzstrasse 16**
**D-8000 München 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 108 285 B1

**Description**

The present invention relates to antiviral purine derivatives containing an acyclic chain in the 9-position.

U.K. Patent Specification No. 1523865 describes a broad class of purine derivatives containing an acyclic side chain in the 9-position. These purine derivatives have been found to have antiviral activity against various classes of DNA viruses particularly against herpes viruses such as herpes simplex.

Among these derivatives, 9-(2-hydroxyethoxymethyl)guanine (otherwise known as acyclovir) has been found to have particularly good activity against herpes viruses such as herpes simplex. However, while acyclovir has been found to be especially effective upon topical or parenteral administration, it is only moderately well absorbed upon oral administration with corresponding levels of drug in the plasma. It will be appreciated that when one is treating an internal disorder by oral administration of a drug, it is clearly desirable that the drug should be well absorbed from the gastro-intestinal tract with resulting high plasma levels.

We have now made the surprising discovery that a purine derivative which is characterised by the presence of a hydrogen atom in the 6-position of the purine nucleus, namely 6-deoxyacyclovir, i.e. 2-amino-9-(2-hydroxyethoxymethyl)purine, the 6-hydrogen analogue of acyclovir, can be readily converted *in vivo* by the action of enzymes of the molybdo-flavo-protein type (especially xanthine oxidase/dehydrogenase or aldehyde oxidase) into the corresponding 6-hydroxy purine derivative having antiviral activity. Furthermore, from experiments in rats, we have found that oral administration of such a 6-hydrogen derivative results in efficient absorption from the gastro-intestinal tract and high plasma levels of the corresponding 6-hydroxy compound, formed by enzymatic conversion of the 6-hydrogen compound. Also, for example, 6-deoxyacyclovir is also considerably more soluble in water than acyclovir, the former compound having a solubility of 50mg/ml and the latter having a solubility of 1.23mg/ml at 25°C. This improved water-solubility enables 6-deoxyacyclovir to be used in a greater variety of aqueous pharmaceutical formulations which require some solubilisation of the drug.

The above-mentioned 6-hydrogen purine derivative may be represented by the formula

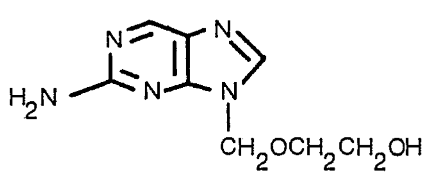

(I)

and physiologically acceptable salts thereof.

Salts of the compound of formula (I) which may be conveniently used in therapy include physiologically acceptable salts of organic acids such as lactic, acetic, malic or p-toluenesulphonic acid as well as physiologically acceptable salts of mineral acids such as hydrochloric or sulphuric acid.

The discovery that the 6-hydrogen purine above can be readily converted into its corresponding 6-hydroxy analogue is surprising since in previous studies with xanthine oxidase from bovine milk (H. Lettre et al (1967) Biochem. Pharmacol., *16*, 1747—1755; T. A. Krenitsky et al (1972) Arch. Biophys., *150*, 585—599), it was shown that 9-substitution obliterates or greatly diminishes the rate at which a variety of purines are oxidised. In view of these observations, it was surprising to find that this enzyme oxidised for example 6-deoxyacyclovir, a 9-substituted derivative of 2-aminopurine, at a faster rate than the 9-unsubstituted purine, as we have established from enzyme studies.

The high level of absorption of the compound of formula (I) from the gastro-intestinal tract renders the compound especially useful when oral administration of the compound is desired, e.g. in the treatment of diseases caused by various DNA viruses, such as herpes infections for example herpes simplex, varicella or zoster, cytomegalovirus as well as diseases caused by hepatitis B or Epstein-Barr virus. The compound of formula (I) can also be used for the treatment or prophylaxis of papilloma or wart virus infections. In addition to its use in human medical therapy the compounds of formula (I) can be administered to other animals for the treatment or prophylaxis of viral diseases, e.g. in other mammals.

According to a further feature of the present invention we provide a compound of formula (I) and physiologically acceptable salts thereof for use in the treatment or prophylaxis of a viral disease in an animal, e.g. a mammal such as man.

The compound of formula (I) and the physiologically acceptable salts thereof (hereafter collectively referred to as the active ingredients) may be administered by any route appropriate to the condition to be treated, suitable routes including oral, rectal, nasal, topical (including buccal and sublingual), vaginal and parenteral (including subcutaneous, intramuscular, intravenous, intradermal, intrathecal and epidural). It will be appreciated that the preferred route may vary with for example the condition of the recipient.

For each of the above-indicated utilities and indications the amount required of an active ingredient (as above defined) will depend upon a number of factors including the severity of the condition to be treated and the identity of the recipient and will ultimately be at the discretion of the attendant physician or

veterinarian. In general however, for each of these utilities and indications, a suitable, effective dose will be in the range 0.1 to 250mg per kilogram bodyweight of recipient per day, preferably in the range 1 to 100mg per kilogram bodyweight per day and most preferably in the range 5 to 20mg per kilogram bodyweight per day; an optimum dose is about 10mg per kilogram bodyweight per day. (Unless otherwise indicated all weights of active ingredient are calculated as the parent compound of formula (I): for salts thereof the figures would be increased proportionately). The desired dose is preferably presented as two, three, four or more sub-doses administered at appropriate intervals throughout the day. These sub-doses may be administered in unit dosage forms, for example, containing 10 to 1000mg, preferably 20 to 500mg and most preferably 100 to 400mg of active ingredient per unit dosage form.

While it is possible for the active ingredients to be administered alone it is preferable to present them as pharmaceutical formulations. The formulations, both for veterinary and for human use, of the present invention comprise at least one active ingredient, as above defined, together with one or more acceptable carriers therefor and optionally other therapeutic ingredients. The carrier(s) must be "acceptable" in the sense of being compatible with the other ingredients of the formulation and not deleterious to the recipient thereof.

The formulations include those suitable for oral, rectal, nasal, topical (including buccal and sublingual), vaginal or parenteral (including subcutaneous, intramuscular, intravenous, intradermal, intrathecal and epidural) administration. The formulations may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

For infections of the eye or other external tissues e.g. mouth and skin, the formulations are preferably applied as a topical ointment or cream containing the active ingredient in an amount of, for example, 0.075 to 20% w/w, preferably 0.2 to 15% w/w and most preferably 0.5 to 10% w/w. When formulated in an ointment, the active ingredients may be employed with either a paraffinic or a water-miscible ointment base. Alternatively, the active ingredients may be formulated in a cream with an oil-in-water cream base.

If desired, the aqueous phase of the cream base may include, for example, at least 30% w/w of a polyhydric alcohol, i.e. an alcohol having two or more hydroxyl groups such as propylene glycol, butane 1,3-diol, mannitol, sorbitol, glycerol and polyethylene glycol and mixtures thereof. The topical formulations may desirably include a compound which enhances absorption or penetration of the active ingredient through the skin or other affected areas. Examples of such dermal penetration enhancers include dimethyl sulphoxide and related analogues.

The oily phase of the emulsions of this invention may be constituted from known ingredients in a known manner. While the phase may comprise merely an emulsifier (otherwise known as an emulgent), it desirably comprises a mixture of at least one emulsifier with a fat or an oil or with both a fat and an oil. Preferably, a hydrophilic emulsifier is included together with a lipophilic emulsifier which acts as a stabiliser. It is also preferred to include both an oil and a fat. Together, the emulsifier(s) with or without stabiliser(s) make up the so-called emulsifying wax, and the wax together with the oil and/fat make up the so-called emulsifying ointment base which forms the oily dispersed phase of the cream formulations.

Emulgents and emulsion stabilisers suitable for use in the formulation of the present invention include Tween® 60, Span® 80, cetostearyl alcohol, myristyl alcohol, glyceryl mono-stearate and sodium lauryl sulphate.

The choice of suitable oils or fats for the formulation is based on achieving the desired cosmetic properties, since the solubility of the active compound in most oils likely to be used in pharmaceutical emulsion formulations is very low. Thus the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other

mineral oils can be used.

Formulations suitable for topical administration to the eye also include eye drops wherein the active ingredient is dissolved or suspended in a suitable carrier, especially an aqueous solvent for the active ingredient. The active ingredient is preferably present in such formulations in a concentration of 0.5 to 20%, advantageously 0.5 to 10% particularly about 1.5% w/w.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as for example a nasal spray or as nasal drops, include aqueous or oily solutions of the active ingredient.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described. Preferred unit dosage formulations are those containing a daily dose or unit daily sub-dose, as herein above recited, or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

The present invention further provides veterinary compositions comprising at least one active ingredient as above defined together with a veterinary carrier therefor.

Veterinary carriers are materials useful for the purpose of administering the composition and may be solid, liquid or gaseous materials which are otherwise inert or acceptable in the veterinary art and are compatible with the active ingredient. These veterinary compositions may be administered orally, parenterally or by any other desired route.

For oral administration the compositions can be in the form of a tablet, granule, drench, paste, cachet, capsule or feed supplement. Granules may be made by the well known techniques of wet granulation, precompression or slugging. They can be administered to animals in an inert liquid vehicle so as to form a drench, or in a suspension with water or oil base. Preferably further accessory ingredients such as a dispensing agent are included. These formulations preferably contain from 15 to 85% of the active ingredient.

A paste may be formulated by suspending the active ingredient in a liquid diluent. A stiffening or thickening agent may be included together with a wetting agent or a humectant if the liquid diluent is water. If an emulsion paste is needed then one or more surface active agents should desirably be included. From 25 to 80% by weight of these paste formulations may comprise the active ingredient.

In feed supplements the active ingredient is generally present in large amounts relative to the accessory ingredients, and the supplements may be added directly or after intermediate blending or dilution. Examples of accessory ingredients for such formulations include solid, orally ingestible carriers such as corn meal, soya flour, wheat shorts, soya grits, edible vegetable materials and fermentation residues. The active ingredient is usually incorporated in one or more of the accessory ingredients and intimately and uniformly dispersed by grinding, tumbling or stirring with conventional apparatus. Formulations containing 1 to 90% by weight of the active ingredient are suitable for adding to feeds.

For the treatment of herpes infections in horses, an oral or parenteral dose of from 0.1 to 250mg per kg body weight per day, preferably from 2 to 100mg per kg per day may be required. The dose may be split up into discrete units administered at regular intervals during the day, and repeated daily for up to 14 days or until the infection is cleared. For viral infections in other animals the dose may vary depending on the size and metabolism of the animal. The compositions may be administered in unit dosage form, such as a tablet a few times daily in the amount of 10 to 1000mg per unit dose.

The compound of formula (I) and its physiologically acceptable salts may be prepared in conventional manner by analogous processes for preparing compounds of similar structure, such as those methods described in U.K. Patent Specification No. 1523865.

The present invention further provides, in particular, a process for the preparation of the compound of

formula (I) and physiologically acceptable salts thereof which comprises:—

a) converting a compound of formula

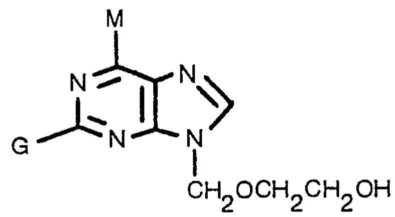

(II)

wherein G represents

(i) an azide group by catalytic hydrogenation of the azide group to an amino group; or

(ii) a halogen atom or an alkylthio or alkylsulphonyl group by aminolysis of the group using ammonia; and where M in formula (II) represents a halogen atom or a mercapto or thio group by conversion of this group into a hydrogen atom.

b) reacting a compound of formula

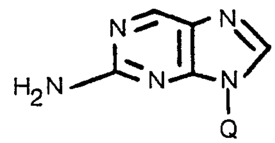

(III)

(wherein Q represents a leaving atom or group) with a compound of formula

$$ACH_2OCH_2CH_2B \quad (IV)$$

(wherein A represents a leaving group or atom and B represents an optionally protected hydroxy group);

c) treatment of a compound of formula

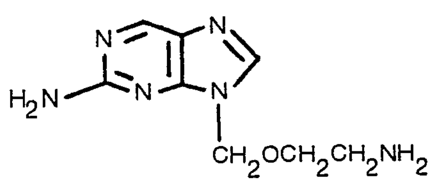

(V)

with a nitrite in an acidic medium to form the compound of formula (I) or a physiologically acceptable salt or ester thereof, and optionally effecting one or more of the following conversions, in any desired sequence:—

(i) where the resulting product is a base, converting the said base into a physiologically acceptable acid addition salt thereof;

(ii) where the resulting product is an acid addition salt, converting the said salt into the parent base; and/or

(iii) where the resulting product is an ester of the compound of formula (I) converting the said ester into the parent compound of formula (I) or a physiologically acceptable salt thereof.

Conversion of a compound of formula (II) into the compound of formula (I), by method a) can be achieved by various conventional means. For example G may represent an azide group which can be reduced to an amino group by catalytic hydrogenation using a suitable catalyst such as palladium. Alternatively, G may represent a halogen atom or an alkylthio or alkylsulphonyl group which can be converted to an amino group by aminolysis using for example ammonia. M may represent a halogen e.g. chlorine, atom or a mercapto or thio (S <) group which can be converted into a hydrogen atom in conventional manner. In the case where M represents a thio group, this conversion may be effected using a compound of formula (II) in which any amino or hydroxyl groups are optionally blocked by acyl groups, the conversion being effected using a Raney nickel catalyst, e.g. in a basic medium which additionally removes the amino and/or hydroxy blocking groups.

Appropriate blocking groups may be selected for example from acyl groups such as $C_1$—$C_4$ alkanoyl group e.g. acetyl or pivaloyl, or aroyl groups, arylmethyl groups or tri $C_{1-4}$ alkylsilyl groups. Arylmethyl blocking groups may be removed for example by hydrogenation in the presence of Raney Nickel or palladium catalyst or by the use of sodium in liquid ammonia. Acyl blocking groups may be removed for example by hydrolysis using for example an amine in an aqueous medium. Trialkylsilyl blocking groups may be removed by alcohol or aqueous ammonia or by alcoholysis.

These processes together with other conventional processes are described in Fused Pyrimidines, Part

II, Purines Ed. by D. J. Brown (1971), Wiley-Interscience.

In process (b), the group Q in formula (III) may for example represent a hydrogen atom; an acyl group, e.g. a $C_{1-4}$alkanoyl group such as an acetyl group or an aroyl group such as a benzoyl group; or a tri-$C_{1-4}$alkylsilyl group such as a trimethylsilyl group. The group A in formula (IV) may for example represent a halogen atom (e.g. chlorine) or an acyloxy group wherein the acyl moiety may be for example a $C_{1-4}$alkanoyl group such as acetyl, or an aroyl group such as benzoyl. The reaction may be conveniently effected in a strong polar solvent such as dimethylformamide or hexamethylphosphoramide, advantageously in the presence of a base such as triethylamine or potassium carbonate. Alternatively, a thermal condensation may be effected by heating the compounds of formulae (III) and (IV) in the presence of a catalytic amount of a strong acid, e.g. sulphuric acid.

In process c) the conversion is advantageously effected by treatment of the amino compound of formula (V) with a nitrite, e.g. sodium nitrite, in an acidic medium.

The starting materials employed in the processes described above may be prepared in conventional manner, e.g. in accordance with the processes described in the above-mentioned UK Patent Specification 1523865.

The following Examples illustrate the present invention.

## Example 1

2-Amino-9-(2-hydroxyethoxymethyl)-9H-purine

A mixture of 2.48g (7.13mM) of 2-amino-6-chloro-9-(2-benzoyloxyethoxymethyl)purine, 250ml of absolute ethanol, 1.9ml of triethylamine and 0.6g of 5% palladium on charcoal was shaken under hydrogen at an initial pressure of 3.4 bar (50 p.s.i.) at room temperature for twenty hours. The mixture was filtered, 0.265g of fresh palladium catalyst oand 1.9ml of triethylamine were added and the mixture shaken under hydrogen for an additional 16 hours.

The ethanolic solution, after filtration through a pad of Celite® was evaporated *in vacuo*, and the resulting white solid extracted with boiling benzene several times. The benzene extracts were concentrated, combined with 20ml of 40% aqueous methylamine and 20ml of methanol and allowed to evaporate in an open flask on a steam bath to dryness. The resulting mixture was triturated with ether to remove the N-methylbenzamide and then recrystallised from 100% ethanol to yield 2-amino-9-(2-hydroxyethoxymethyl)-9H-purine as analytically pure white granules, m.p.=186—187.5°C.

## Example 2

a) 2-Acetamido-9-acetyl-6-mercapto-9H-purine

A mixture of thioguanine (54.0g), acetic anhydride (250ml) and p-toluenesulfonic acid (2.0g) was heated at reflux overnight. The reaction mixture was cooled and the resulting solid removed by filtration and thoroughly washed with acetone to give 2-acetamido-9-acetyl-6-mercapto-9H-purine (62.3g) as a brown solid, nmr spectrum was consistent with the assigned structure.

b) 2-Acetamido-9-(2-acetoxyethoxymethyl)-6-mercepto-9H-purine

To a mixture of 2-acetamido-9-acetyl-6-mercapto-9H-purine (30.0g) and p-toluenesulfonic acid (2.0g) in toluene (150ml) was added dropwise over 4 hours at 90—95°C 2-oxa-1,4-butanediol diacetate (40.0g). After 10 hours the reaction mixture was cooled, and the resulting solid removed by filtration and washed with acetone. It was recrystallised from dimethylformamide/acetonitrile (I/I) with decolourisation using activated charcoal to give 2-acetamideo-9-(2-acetoxyethoxymethyl)-6-mercapto-9H-purine (25.6g), m.p. 205—206°C as a yellow solid.

c) 2-Amino-9-(2-hydroxyethoxymethyl)-9H-purine

2-Acetamido-9-(2-acetoxyethoxymethyl)-6-mercapto-9H-purine (0.50g, 1.53mmole), freshly prepared W-2 Raney Nickel (2g) and 58% ammonium hydroxide (25ml) were heated at reflux for 2 hours. The catalyst was filtered off and the filtrate concentrated to 5ml. Acetone (20ml) was added and the resultant precipitate was filtered and dried to give 0.21g (65%) of white solid, m.p. 188—189°, consistent by TLC and NMR with an authentic sample.

## Example 3

a) 9-(2-Phthalimidoethoxymethyl)-2-aminopurine

A mixture of 1.35g (10.0mM) of 2-aminopurine and 0.44g (11.0mM) of 60% sodium hydride-oil dispersion in 50ml of dry dimethylformide was stirred at room temperature for 35 minutes. To the mixture was added 2.63g (11.0mM) of 2-phthalimidoethoxymethyl chloride and the stirring continued at room temperature for five hours. An additional 156mg (3.9mM) of sodium hydride dispersion and 0.94g (3.9mM) of 2-phthalimidoethoxymethyl chloride were added and the reaction mixture stirred at room temperature for 20 hr.

The mixture was evaporated *in vacuo* and the residue partitioned between chloroform and water. The aqueous phase was extracted twice more with chloroform and the combined chloroform extracts washed with water and dried over sodium sulfate. Evaporation of the dried extracts and chromatography on silica gel, gave an elution with 10% methanol in dichloromethane, 1.7g of 9-(2-phthalimidoethoxymethyl)-2-aminopurine.

b) 9-(2-Aminoethoxymethyl)-2-aminopurine

A mixture of 5.0g (14.7mM) of 9-(2-phthalimidoethoxymethyl)-2-aminopurine, 500ml of ethanol and 15ml of hydrazine (95%) was refluxed with stirring for four hours. The reaction mixture was evaporated *in vacuo* and evaporated twice more with ethanol. The solids were stirred for thirty minutes with 200ml of 5% aqueous acetic acid then the solution was evaporated *in vacuo* to dryness. Two recrystallisations, once from ethanol-ether and once from ethanol gave analytically pure 9-(2-aminoethoxymethyl)-2-aminopurine as the diacetate. Treatment of an aqueous solution of the product with strong OH resin gave the free base, 1.5g on evaporation.

c) 2-Amino-9-(2-hydroxyethoxymethyl)-9H-purine

To a chilled (0°C) solution of 1.0g (4.8mM) of 9-(2-aminoethoxymethyl)-2-aminopurine in 25ml of 0.2 N HCl and 25ml ethanol were added 0.335g (4.86mM) of sodium nitrite in portions over a period of 45 minutes. The mixture was stirred to 0°C for 18hr.

The reaction mixture was evaporated *in vacuo* (bath temp. <30°C) and the residue dissolved in methanol and absorbed on silica gel. The solvent was evaporated off and the powder added to a column prepared for flash chromatography. Elution with 10% methanol in dichloromethane gave on evaporation, the desired 9-(2-hydroxyethoxymethyl)-2-aminopurine. Recrystallisation from ethanol gave analytically pure product.

The following Examples, 4 to 7, illustrate pharmaceutical formulations according to the invention where the active compound is the above compound of formula (I), namely 2-amino-9-(2-hydroxyethoxymethyl)-9H-purine.

| Example 4 | Tablet |
|---|---|
| Active compound | 200mg |
| Lactose | 235mg |
| Starch | 50mg |
| Polyvinylpyrrolidone | 50mg |
| Magnesium stearate | 5mg |
| | 500mg |

Mix the active compound with the lactose and starch and wet granulate with a solution of the polyvinylpyrrolidone. Dry, sift, blend the granules with magnesium stearate and compress.

| Example 5 | Capsule |
|---|---|
| Active compound | 200mg |
| Lactose | 184mg |
| Sodium starch glycollate | 8mg |
| Polyvinylpyrrolidone | 6mg |
| Magnesium stearate | 2mg |

Mix the active compound with the lactose and sodium starch glycollate and wet granulate with a solution of the polyvinylpyrrolidone. Dry, sift, blend the granules with the magnesium stearate and fill into hard gelatin capsules.

| Example 6 | | Cream |
|---|---|---|
| Active compound | | 5.00g |
| Glycerol | | 2.00g |
| Cetostearyl alcohol | | 6.75g |
| Sodium lauryl sulphate | | 0.75g |
| White soft paraffin | | 12.50g |
| Liquid paraffin | | 5.00g |
| Chlorocresol | | 0.10g |
| Purified water | to | 100.00g |

Dissolve the active compound in a mixture of purified water and glycerol and heat to 70°C. Heat the remaining ingredients together at 70°C. Add the two parts together and emulsify. Cool and fill into containers.

| | Example 7 | | Intravenous Injections |
|---|---|---|---|
| A) | Active compound | | 200mg |
| | Sodium hydroxide solution | | q.s. to pH 7.0 to 7.5 |
| | Water for injections | to | 5.0ml |

Dissolve the active compound in part of the water for injections. Adjust the pH with the sodium hydroxide solution and make up to volume with additional water for injections. Under aseptic conditions, sterilise the solution by filtration, fill into sterile ampoules and seal the ampoules.

| | | | |
|---|---|---|---|
| B) | Active compound | | 100mg |
| | Sodium hydroxide solution | | q.s. to pH 7.0 to 7.5 |
| | Mannitol | | 125mg |
| | Water for injections | to | 2.5ml |

Dissolve the active compound and mannitol in part of the water for injections. Adjust the pH with the sodium hydroxide solution and make up to volume with additional water for injections. Under aseptic conditions, sterilise the solution by filtration, fill into sterile vials and remove the water by freeze-drying. Seal the vials under an atmosphere of nitrogen and close with a sterile stopper and aluminium collar.

Biological Activity

The following experiments carried out to determine the urinary excretion and plasma levels of acyclovir after oral dosing of rats with acyclovir, 6-deoxyacyclovir and the 6-amino analogue thereof i.e. 2,6-diamino-9-(2-hydroxyethoxymethyl)-purine, hereinafter referred to as aminoacyclovir. The latter compound is a prodrug of acyclovir (Good S.S. and de Miranda P., Fed. Proc. (1982), *42*, 1733) which depends on adenosine deaminase for conversion to acyclovir *in vivo*.

Procedure

Long Evans male rats were dosed by intragastric needle with the drug and placed in metabolic cages which separated urine from faeces. The collected urine and plasma samples were analyzed for acyclovir content by a radioimmunoassay. It was demonstrated that neither aminoacyclovir nor 6-deoxyacyclovir cross-reacted with the antisera used in the assay.

Results

The results are given below in Tables I and II.

TABLE I

The urinary excretion of acyclovir after oral dosing of rats with acyclovir, aminoacyclovir and 6-deoxyacyclovir.

| Dose mg/kg | Urine samples | %Dose Excreted as Acyclovir (No. of Animals) Acyclovir | Aminoacyclovir | 6-Deoxyacyclovir |
|---|---|---|---|---|
| 5 | 0—24hr | — | 24.7(2) | 67.0(1) |
| | 24—48hr | — | 0.4(2) | 0.2(1) |
| 20 | 0.24hr | — | 24.8(2) | 69.1(1) |
| | 24—48hr | — | 0.3(2) | 0.3(1) |
| 25 | 0.48hr | 19.2(4) | — | — |

TABLE II

A comparison of the plasma levels of acyclovir achieved after oral dosing of rats with deoxyacyclovir and with aminoacyclovir.

| Hr after a 20 mg/kg dose | μM Acyclovir Concentrations in Plasma | | |
|---|---|---|---|
| | Aminoacyclovir | 6-Deoxyacyclovir | |
| | Rat 1 | Rat 2 | Rat 3 |
| 0.5 | 2.75 | | 30 |
| 1 | 3.03 | 16.6 | 18.6 |
| 2 | 2.48 | 4.96 | 17.5 |
| 4 | 0.93 | 1.00 | 6.7 |
| 6 | 0.34 | 0.41 | 4.06 |
| 20 | 0.1 | 0.01 | 0.25 |

Toxicity Study

Two male and 2 female beagle dogs were given 6-deoxyacyclovir per os (ounce daily for 5 consecutive days) at a dose level of 40mg/kg/day. There were no effects in 1 male and 1 female sacrificed at the end of the treatment period or in 1 male and 1 female sacrificed after a 2-week post-dose period.

Antiviral Activity

70 CD1 mice were infected with 300 $LD_{50}$ of the ICI strain of herpes simplex virus Type 1 administered in a dose of 0.025ml intracerebrally. The mice were divided into groups of 10, one group acting as untreated virus controls and the other groups receiving respectively (A) acyclovir at 100mg/kg/dose (i) subcutaneously (s.c.) (ii) orally (iii) intraperitoneally (i.p.) (B) the compound of Example 1 (6-deoxyacyclovir) at 100mg/kg/dose i) subcutaneously (s.c.) ii) orally iii) intraperitoneally (i.p.). The treatment groups received dosages of the drug 2—3 hours after infection and twice daily for 4 days. Observations were made over 14 days survival times were recorded. The results are shown below:

RESULTS

| Treatment group | Survival Times (Days) | Mean Reciprocal Survival times |
|---|---|---|
| Virus Controls | 4+2 1+2 5+3 | 0.407 |
| Acyclovir (s.c.) | 3+3 4+4 2+4 1+6 | 0.261 |
| 6-Deoxyacyclovir (s.c.) | 1+2 3+3 1+4 2+4 2+5 | 0.289 |
| Acyclovir (oral) | 2+2 3+3 5+4 | 0.325 |
| 6-Deoxyacyclovir (oral) | 1+2 1+4 2+5 3+6 1+8 1 survivor | 0.186 |
| Acyclovir (i.p.) | 2+2 2+4 2+5 2+7 1 survivor | 0.242 |
| 6-Deoxyacyclovir (i.p.) | 1+2 2+3 1+4 1+4 4+6 1+8 | 0.233 |

The above results demonstrate that 6-deoxyacyclovir has a more effective action than acyclovir when administered orally.

**Claims**

1. 2-Amino-9-(2-hydroxyethoxymethyl)-9H-purine.
2. Physiologically acceptable salts of 2-amino-9-(2-hydroxyethoxymethyl)-9H-purine.
3. Pharmaceutical formulations comprising 2-amino-9-(2-hydroxyethoxymethyl)-9H-purine or a physiologically acceptable salt thereof together with one or more pharmaceutically acceptable carriers therefor.
4. Pharmaceutical formulations as claimed in claim 3 adapted for oral administration.
5. Pharmaceutical formulations as claimed in claim 4 in the form of tablets, capsules, powders, granules; solutions or suspensions in an aqueous or non-aqueous liquid; or oil-in-water or water-in-oil liquid emulsions.
6. Pharmaceutical formulations as claimed in claim 4 in the form of dosage units.
7. Pharmaceutical formulations as claimed in claim 6 wherein each dosage unit contains 10 to 1000mg of 2-amino-9-(2-hydroxyethoxymethyl)-9H-purine or a physiologically acceptable salt thereof.
8. Pharmaceutical formulations as claimed in claim 7 wherein each dosage unit contains 20 to 500mg of 2-amino-9-(2-hydroxyethoxymethyl)-9H-purine or a physiologically acceptable salt thereof.
9. 2-Amino-9-(2-hydroxyethoxymethyl)-9H-purine or a physiologically acceptable salt thereof for use in the treatment or prophylaxis of a viral disease in an animal.
10. 2-Amino-9-(2-hydroxyethoxymethyl)-9H-purine or a physiologically acceptable salt thereof for use in the treatment or prophylaxis of a herpes viral disease in man.
11. 2-Amino-9-(2-hydroxyethoxymethyl)-9H-purine or a physiologically acceptable salt thereof for use in the treatment or prophylaxis of a herpes zoster infection in man.
12. 2-Amino-9-(2-hydroxyethoxymethyl)-9H-purine or a physiologically acceptable salt thereof for use in generating acyclovir *in vivo* when the said purine is administered to an animal.
13. 2-Amino-9-(2-hydroxyethoxymethyl)-9H-purine or a physiologically acceptable salt thereof for use in generating acyclovir *in vivo* when the said purine is administered to an animal by the oral route.
14. A process for the preparation of the compound of formula (I) (as defined in claim 1) and physiologically acceptable salts thereof which comprises:—

a) converting a compound of formula (II)

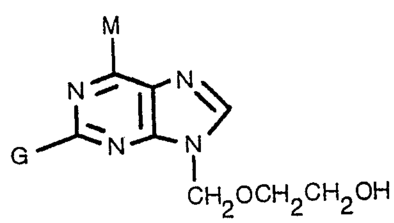

(II)

wherein G represents

(i) an azide group by catalytic hydrogenation of the azide group to an amino group; or

(ii) a halogen atom or an alkylthio or alkylsulphonyl group by aminolysis of the group using ammonia; and where M in formula (II) represents a halogen atom or a mercapto or thio group by conversion of this group into a hydrogen atom.

b) reacting a compound of formula

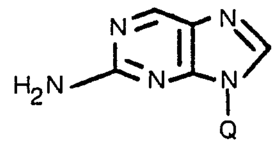

(III)

(wherein Q represent a leaving atom or group) with a compound of formula

$$ACH_2OCH_2CH_2B \quad (IV)$$

(wherein A represents a leaving group or atom and B represents a optionally protected hydroxy group);

c) treatment of a compound of formula (V)

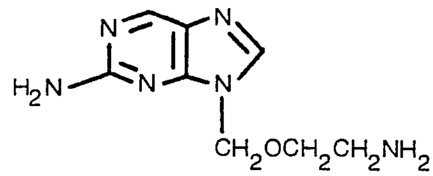

(V)

with a nitrile in an acidic medium to form the compound of formula (I) or a physiologically acceptable salt or ester thereof, and optionally effecting one or more of the following conversions, in any desired sequence:

i) where the resulting product is a base, converting the said base into a physiologically acceptable acid addition salt thereof;

ii) where the resulting product is an acid addition salt, converting the said salt into the parent base; and/or

iii) where the resulting product is an ester of the compound of formula (I) converting the said ester into the parent compound of formula (I) or a physiologically acceptable salt thereof.

**Patentansprüche**

1. 2-Amino-9-(2-hydroxyethoxymethyl)-9H-purin.

2. Physiologisch annehmbare Salze von 2-Amino-9-(2-hydroxyethoxymethyl)-9H-purin.

3. Pharmazeutische Formulierungen enthaltend 2-Amino-9-(2-hydroxyethoxymethyl)-9H-purin oder ein physiologisch annehmbares Salz davon zusammen mit einem oder mehreren pharmazeutisch annehmbaren Trägermaterialien.

4. Pharmazeutische Formulierungen nach Anspruch 3, angepaßt für die orale Verabreichung.

5. Pharmazeutische Formulierungen nach Anspruch 4 in Form von Tabletten, Kapseln, Pulvern, Granula; Lösungen oder Suspensionen in einer wäßrigen oder nicht wäßrigen Flüssigkeit, oder Öl-in-Wasser oder Wasser-in-Öl Flüssigemulsionen.

6. Pharmazeutische Formulierungen nach Anspruch 4 in Form von Einzeldosen.

7. Pharmazeutische Formulierungen nach Anspruch 6, worin jede Einzeldosis 10 bis 1000 mg 2-Amino-9-(2-hydroxyethoxymethyl)-9H-purin oder ein physiologisch annehmbares Salz davon enthält.

8. Pharmazeutische Formulierungen nach Anspruch 7, worin jede Einzeldosis 20 bis 500 mg 2-Amino-9-(2-hydroxyethoxymethyl)-9H-purin oder ein physiologisch annehmbares Salz davon enthält.

9. 2-Amino-9-(2-hydroxyethoxymethyl)-9H-purin oder ein physiologisch annehmbares Salz davon zur Verwendung in der Behandlung oder Prophylaxe einer viralen Erkrankung bei einem Tier.

10. 2-Amino-9-(2-hydroxyethoxymethyl)-9H-purin oder ein physiologisch annehmbares Salz davon zur Verwendung in der Behandlung oder Prophylaxe einer Herpes-Viruserkrankung beim Menschen.

11. 2-Amino-9-(2-hydroxyethoxymethyl)-9H-purin oder ein physiologisch annehmbares Salz davon zur Verwendung in der Behandlung oder Prophylaxe einer Herpes-Zosterinfektion beim Menschen.

12. 2-Amino-9-(2-hydroxyethoxymethyl)-9H-purin oder ein physiologisch annehmbares Salz davon zur Verwendung in der Erzeugung von Acyclovir in vivo, wenn das Purin einem Tier verabreicht wird.

13. 2-Amino-9-(2-hydroxyethoxymethyl)-9H-purin oder ein physiologisch annehmbares Salz davon zur Verwendung in der Erzeugung von Acyclovir in vivo, wenn das Purin einem Tier oral verabreicht wird.

14. Verfahren zur Herstellung einer Verbindung der Formel (I) (wie in Anspruch 1 definiert) und physiologisch annehmbarer Salze davon bei dem man:

a) eine Verbindung der Formel (II)

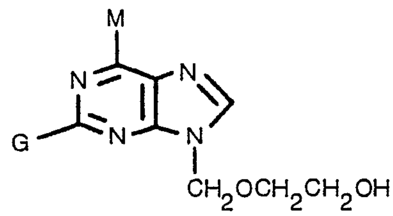

(II)

umwandelt, worin G

(i) eine Azidgruppe bedeutet, durch katalytische Hydrierung der Azidgruppe in eine Aminogruppe, oder

(ii) ein Halogenatom oder eine Alkylthio-oder Alkylsulfonylgruppe bedeutet, durch Aminolyse der Gruppe unter Verwendung von Ammoniak;

und wenn M in der Formel (II) ein Halogenatom oder eine Mercapto- oder Thiogruppe bedeutet durch Überführung dieser Gruppe in ein Wasserstoffatom.

b) eine Verbindung der Formel

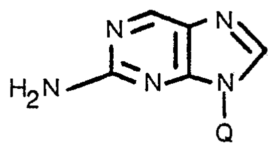

(III)

(worin Q ein austretendes Atom oder Gruppe bedeutet) mit einer Verbindung der Formel

$$ACH_2OCH_2CH_2B \quad (IV)$$

(worin A eine austretende Gruppe oder Atom bedeutet und B eine gegebenfalls geschützte Hydroxygruppe bedeutet), umsetzt;

c) eine Verbindung der Formel (V)

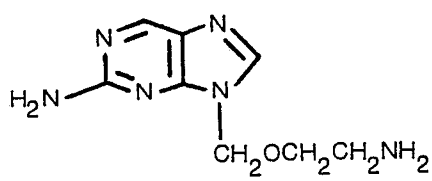

(V)

mit einem Nitrit in einem sauren Medium zur Bildung einer Verbindung der Formel (I) oder eines physiologisch annehmbaren Salzes oder Esters davon behandelt und gegebenenfalls eine oder mehrere der folgenden Umwandlungen in jeder gewünschten Reihenfolge durchführt:

i) wenn das erhaltene Produkt eine Base ist, Überführung der Base in das physiologisch annehmbare Säureadditionssalz;

ii) wenn das erhaltene Produkt ein Säureadditionssalz ist, Überführung des Salzes in die Stammbase, und/oder.

iii) wenn das erhaltene Produkt ein Ester der Verbindung der Formel (I) ist, Überführung des Esters in die Stammverbindung der Formel (I) oder ein physiologisch annehmbares Salz.

**Revendications**

1. La 2-amino-9-(2-hydroxyéthoxyméthyl)-9H-purine.

2. Sels physiologiquement acceptables de la 2-amino-9-(2-hydroxyéthoxyméthyl)-9H-purine.

3. Compositions pharmaceutiques comprenant de la 2-amino-9-(2-hydroxyéthoxyméthyl)-9H-purine ou un sel physiologiquement acceptable de celle-ci conjointement avec un ou plusiers excipients pharmaceutiquement acceptables.

4. Compositions pharmaceutiques suivant la revendication 3, propres à l'administration par voie orale.

5. Compositions pharmaceutiques suivant la revendication 4, sous forme de comprimés, de capsules, de poudres, de granules, de solutions ou suspensions dans un liquide aqueux ou non aqueux ou d'émulsions liquides huile-dans-eau ou eau-dans-huile.

6. Compositions pharmaceutiques suivant la revendication 4, sous la forme d'unités dosées.

7. Compositions pharmaceutiques suivant la revendication 6, dans lesquelles chaque unité dosée contient 10 à 1000 mg de 2-amino-9-(2-hydroxyéthoxyméthyl)-9H-purine ou d'un sel physiologiquement acceptable de celle-ci.

8. Compositions pharmaceutiques suivant la revendication 7, dans lesquelles chaque unité dosée contient 20 à 500 mg de 2-amino-9-(2-hydroxyéthoxyméthyl)-9H-purine ou d'un sel physiologiquement acceptable de celle-ci.

9. La 2-amino-9-(2-hydroxyéthoxyméthyl)-9H-purine ou un sel physiologiquement acceptable de celle-ci à utiliser pour le traitement ou la prophylaxie d'une affection virale chez un animal.

10. La 2-amino-9-(2-hydroxyéthoxyméthyl)-9H-purine ou un sel physiologiquement accep table de celle-ci à utiliser pour le traitement ou la prophylaxie d'une infection par le virus de l'herpès chez l'homme.

11. La 2-amino-9-(2-hydroxyéthoxyméthyl)-9H-purine ou un sel physiologiquement acceptable de celle-ci à utiliser pour le traitement ou la prophylaxie d'une infection par herpes zoster chez l'homme.

12. La 2-amino-9-(2-hydroxyéthoxyméthyl)-9H-purine ou un sel physiologiquement acceptable de celle-ci à utiliser pour engendrer l'acyclovir *in vivo* lorsque cette purine est administrée à un animal.

13. La 2-amino-9-(2-hydroxyéthoxyméthyl)-9H-purine ou un sel physiologiquement acceptable de celle-ci à utiliser pour engendrer l'acyclovir *in vivo* lorsque cette purine est administrée à l'animal par voie orale.

14. Procédé de préparation du composé de formule (I) (tel que défini dans la revendication 1) et de ses sels physiologiquement acceptables, qui comprend:

a) la conversion d'un composé de formula

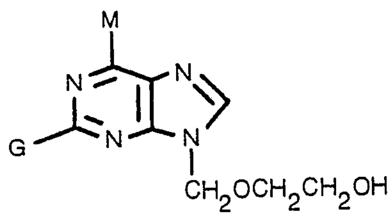

(II)

où G représente

(i) un radical azido, par hydrogénation catalytique du radical azido en un radical amino; ou

(ii) un atome d'halogène ou un radical alcoylthio ou alcoylsulfonyle, par aminolyse du radical au moyen d'ammoniac;

et lorsque M dans la formule (II) représente un atome d'halogène ou un radical mercapto ou thio, par conversion de ce radical en un atome d'hydrogène;

b) la réaction d'un composé de formule

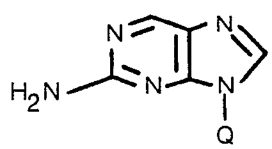

(III)

(où Q représente un atome ou radical partant) avec un composé de formule

$$ACH_2OCH_2CH_2B \qquad (IV)$$

(où A représente un radical ou atome partant et B représente un radical hydroxyle éventuellement protégé);

c) le traitement d'un composé de formule

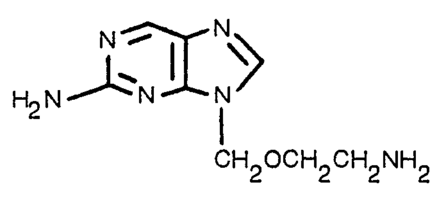

(V)

au moyen d'un nitrite dans un milieu acide pour former le composé de formule (I) ou un sel ou ester physiologiquement acceptable de celui-ci, et éventuellement l'exécution d'une ou plusieurs des conversions suivantes, dans tout ordre souhaité:

(i) lorsque le produit résultant est une base, la conversion de cette base en un sel d'addition d'acide physiologiquement acceptable de celle-ci;

(ii) lorsque le produit résultant est un sel d'addition d'acide, la conversion de ce sel en la base dont il est issu; et/ou

(iii) lorsque le produit résultant est un ester du composé de formule (I), la conversion de cet ester en le composé de formule (I) dont il est issu ou en un sel physiologiquement acceptable de celui-ci.